# EUROPEAN PATENT SPECIFICATION

(11) **EP 1 469 834 B1**
(45) Date of publication and mention of the grant of the patent: **03.11.2010**
(21) Application number: 03705420.2
(22) Date of filing: 29.01.2003
(51) Int. Cl.: A61K 9/16

(54) **MULTI-STAGE ORAL DRUG CONTROLLED-RELEASE SYSTEM**
MEHRSTUFIGES ORALES ARZNEIMITTELSYSTEM MIT KONTROLLIERTER FREISETZUNG
SYSTEME DE LIBERATION CONTROLEE D'UN MEDICAMENT ORAL A ETAPES MULTIPLES

(30) Priority: 01.02.2002 KR 2002005858
(43) Date of publication of application: 27.10.2004
(73) Proprietor: PACIFIC CORPORATION, Seoul 140-777 (KR)
(72) Inventor: PARK, Jin Woo, 157-744 Seoul (KR); BAE, Joon Ho, 156-761 Seoul (KR); KIM, Jung Ju, 102-601, Dongbo Apt., Yongin-si, 449-759 Gyeonggi-do (KR)
(74) Representative: Jones, Nicholas Andrew
(86) International application number: PCT/KR2003/000200
(87) International publication number: WO 2003/063834

(56) References cited:
- WO-A-00/76481
- WO-A-93/00889
- US-A- 2 996 431
- US-A- 4 880 830
- US-A- 5 273 760
- US-A- 5 582 837
- US-A- 5 811 126
- US-B1- 6 294 195
- AOKI S. ET AL: 'Preparation of a novel type of controlled-release carrier and evaluation of drug release from the matrix tablet and its physical-properties' INTERNATIONAL JOURNAL OF PHARMACEUTICS, USA vol. 85, no. 1-3, 1992, pages 29 - 38, XP002903369

## Description

### Technical Field

The present invention relates to, as a novel oral drug delivery system for controlling drug release, a preparation for maintaining drug concentration in blood at a certain level for prolonged time by allowing the drug to be released by a constant rate through stepwise control of drug release upon the administration of the preparation.

### Background Art

Administration forms capable of controlling drug release become an important part of medication in terms of improved treatment effect, reduction of side effects and patient's convenience. Such controlled-release of drug is accomplished through designing of a system comprising the drug. Controlled-release of drug brings many therapeutic advantages, and the most important point is that blood level of drug can be maintained for long time while minimizing fluctuation of the blood level. Accordingly, allowing drug to be released at a constant rate from a preparation is the most important aspect in controlled-release preparation, and in particular, an amount of drug equivalent to that eliminated from the body should be released from the preparation and continuously absorbed while passing through the gastrointestinal tract.

Controlled-release preparations developed so far can be divided into three types, i.e. a type in which drug-containing particles (granules) are coated, matrix type mainly based on polymers, and a type based on osmotic pressure, and among them, the matrix form tablet has been interested greatly as a drug delivery system for the advantage of easy manufacture. When compared with tablets, because of the size and resultant increase of surface area, granules lead to relatively fast disintegration, resulting in the disadvantage of a short drug-release time in a body.

Most matrix preparations release a drug via diffusion, and regarding with the matrix preparations, various techniques such as introducing water-insoluble coating layer on matrix particles in which drug is dispersed have been developed. In case components of coating layer and the matrix are insoluble in body fluid, diffusion of drug is controlled by the components of coating layer or matrix. Drug release from such preparation occurs by concentration gradient of drug introduced by water penetrated to the preparation. Such type of release shows a tendency of decline in the release rate at the last stage due to the gradual reduction of concentration gradient and the gradual increase of diffusion distance. Accordingly, release rate of drug cannot be maintained at a constant level but gradually reduces as a function of time, finally failing to maintain constant blood level of drug.

Such simple matrix tablets just extend the period of drug release, and exhibit inherent limit of releasing drug by first order kinetics or at a rate of (time)^{0.5}. To maintain constant release rate, attempts to modify the previous matrix formulations have been made. Representative methods are to reduce initial drug release rate by introduction of a coating layer, to induce zero-order release rate by morphological approach to preparation, and to combine said two methods. Another approach is method of maintaining constant release rate by allowing diffusion distance to be reduced as a function of time through using erodible and swelling polymer as a main component of matrix.

Majority of the complements to the matrix preparation via coating were attempted for special object besides the control of release rate, e.g. enteric coated tablet or delayed release of drug in colon. As the best example of morphological approach to preparation, a method of regulating release area by introducing hydrophilic or hydrophobic layer on both sides of drug-containing layer and a method of exposing constant area of the coated tablet can be enumerated.

Matrix formulation mainly consists of a drug and a biocompatible polymer, and in particular, in controlled-release preparation, polymer acts a very important role. Polymer matrix with the characteristic of swelling and erosion consists of swelling layer, diffusion layer and erosion layer, and has the advantage that drug release rate can be regulated at a fixed level based on the moving rates of swelling layer and erosion layer. However, also in case of using erosive polymer, release area deceases with time and this leads to typical matrix release mechanism pattern where release rate decreases with reduction of release area. To correct such drug release pattern, coating layer and a factor capable of controlling swelling were introduced. USP 6,156,343 retarded swelling and initial release by use of polyvinyl alcohol as material for matrix core, and by addition of a salt and introduction of a coating layer.

However, besides the simple erodible polymeric matrix system, non-erodible preparation with coating layer comprising water-insoluble polymer such as lacquer is still defective for time-dependent reduction of drug release, and osmotic preparation is disadvantageous for complicacy of the system and cost problem.

To overcome the declination of drug release with time, DE 1,767,765 developed multi-layer tablets, layers with different concentration of drug, and DE 2,651,176 designed a tablet in which drug concentration can increase from the outer layer towards the center. However, like osmotic preparation, the multi-layer tablet also has some disadvantages, necessity for special facility and complicate manufacture.

USP 4,252,786 designed a preparation in which the core of water-insoluble swelling polymer swells with penetration of water to lead to burst of coating layer. Such pulsitile drug release is desirable for improving bioavailability of a drug whose first pass effect can be saturated, and it was revealed that drug release from the preparation is less sensitive to pH value of GI tract. Such preparation can freely control the delay of initial drug release, yet, drug release after the burst of the coating layer, still, depends on concentration gradient of drug.

USP 4,610,870 (Jain et al.) disclosed a coated tablet showing zero-order release rate. The core of this tablet includes hydroxypropylmethylcellulose and/or methylcellulose, one or more non-swellable binders and/or wax binders, one or more inert fillers or excipients, and one or more lubricant.

USP 4,252,786 by Weiss et al*.* resolved the rapid initial-release problem of swelling and erodible formulation by coating the swelling matrix core with a hydrophobic film coating layer capable of burst. Drug release in this preparation occurs via diffusion through initial non-damaged coating layer, and core expands by continuous penetration of external fluid, leading to burst of the coating layer. Thereafter, the swelling matrix core controls the drug release. Overall drug release is continuous based on such control of initial release, and zero-order release can be achieved.

Though said two patents resolved the problem of non-linear drug release that can occur in swelling and erodible matrix tablet by introducing a coating layer, it is still only simple coated tablet, therefore has failed in overcoming the feature and basic limitations of swelling and erodible matrix. Further, in case of a drug with high water-solubility, it is not effective for prolonged release over 24 hr.

USP Nos. 4,309,404 and 4,248,857 (DeNeale et al.) used carboxypolymethylene as substance for core and introduced seal coating and sugar coating thereon, and USP No. 4,309,405 (Guley et al.) disclosed the similar formulation with the above one, using a combination of hydroxypropylmethylcellulose or hydroxypropylcellulose and hydrophobic polymer as core substance. These two formulations demonstrated zero-order release pattern over 12 hr, yet only after rapid initial drug release for 1 hr.

USP No. 4,610,870 discloses a coated tablet showing zero-order release pattern over 8 to 12 hr, and the coating layer of this tablet inhibits the rapid initial release while being gradually disappeared by swelling of the core layer, and then, drug is released with erosion of the core.

USP No. 5,464,633 introduced compressed layer instead of coating layer to swelling and erodible core matrix tablet in order to modify drug release rate, thereby preventing rapid initial drug release, and at the same time, endowed sustained release effect over prolonged time. In case of such multi-layer tablet, to remove inconvenience of coating for coated tablet, compressed layer was introduced, yet, for formation of compressed layered tablet, special facility and complicate calculation of release area were necessary.

USP No. 6,083,532 compensated for pH dependent behavior of drug solubility by using a combination of pH dependent substance and pH-independent polymer as a constituent of core matrix. Such release-modifying attempts were to make the release uninfluenced by individual patient's physiological condition, and applied as means for maximizing drug action. Such preparations can be applied to only specific group of drugs with specific pH-dependency, and as external fluid penetrates continuously into inside of the matrix, it sensitively reacts to pH within the gastrointestinal tract, thus it is difficult to expect continuously steady drug release.

USP No. 4,610,870 used a mixture of hydroxypropylmethylcellulose and methylcellulose as a gel-forming substance, and introduced a coating layer consisting of hydrophilic and hydrophobic materials on the core tablet. Based on this attempt, a preparation was designed to release procaine hydrochloride by zero-order over 8 to 12 hr.

USP No. 6,068,859 discloses controlled-release preparation of azithromycin where, in order to control time-dependent release of drug, the drug was dispersed and embedded in core matrix comprising four kinds of hydro-colloidal gel-forming substance and drug release was induced by erosion of the matrix, and when needed, a coating layer was introduced. As another method, a mixture of coated particles and particles without coating layer was introduced into a single capsule or tablet to allow drug to be released via release channel formed through the uncoated particles. Such preparations were attempted to achieve a comprehensive continuousness by combining each portions with different characteristics such as multi-particulate system, yet control on each part and mixing ratio thereof is necessary, so large amount of time and effort is required.

WO 99/47128 relates to tablet or capsule as biphasic sustained release delivery system, where particles comprising hydrophilic drug and hydrophobic polymer are dispersed in hydrophilic polymer. This system is applied to drugs with high water-solubility, such as metformin hydrochloride, to lead to increased release time and increased transit time in upper gastrointestinal tract by swelling of the preparation. Though the sustained release is effectively accomplished by controlling drug diffusion via adequate application of discontinuous phase of hydrophilic and hydrophobic substance, still, depends on concentration gradient. Therefore, it shows disadvantage of dumping effect due to rapid initial release and time-dependent reduction of release rate. Therefore, it exhibits sustained release effect for about 10 hr in case of drug with high water-solubility, yet represents typical release profile for a matrix tablet, and thus not effective in terms of long term drug release for more than 24 hr and release rate control.

The conventional techniques as described above experienced difficulty in releasing drug at constant rate for prolonged time due to substantial problems such as time-dependent reduction of drug release area and increase of diffusion distance. In case of preparation based on osmotic pressure, zero-order release can be induced, but it has problem of complicated manufacturing process and high manufacturing cost.

WO00/76481 describes controlled-release oral pharmaceutical compositions containing an active ingredient in granules of an inert lipophilic matrix, dispersed in an outer hydrophilic matrix. WO93/00889 discloses a method for the preparation of compositions with a reduced dissolution rate in the stomach, and a higher dissolution rate in the intestines. This is achieved by the incorporation of a gastroresistant polymer in the pellets/granules.

The present invention makes it the object to provide an oral drug controlled-release preparation with minimized solubility-limit for drug to apply and improved stability, which can release drug at a constant rate for a long time without the disadvantages such as complicate manufacturing process and high manufacturing cost as in osmotic preparation or substantial problems such as time-dependent reduction of drug release area and increase of diffusion distance

### Disclosure of the Invention

The present invention relates to, as a novel oral drug delivery system for control of drug release, a preparation for maintaining drug concentration in blood at a certain level for a prolonged time by allowing the drug to be released by a constant rate through stepwise control of drug release upon the administration of the preparation. More specifically, the present invention relates to controlled-release oral preparation characterized by stepwise release of granules from matrix and of drug from the granules, comprising
(1) granules comprising a drug and a carrier material in size of 0.1 - 1 mm, said carrier material is hydrophobic material in case of a drug with water-solubility of 1 mg/ml or more, while hydrophilic material in case of a drug with water-solubility of less than 1 mg/ml;
(2) a matrix in which said granules are embedded, comprising swelling and erodible polymer(s) and swelling-regulating material(s); and
(3) a release-modifying layer comprising hydrophobic release-modifying polymer, hydrophilic release-modifying polymer, pH-dependent release-modifying polymer or a mixture thereof.

In general, the term "very soluble" is applied to what has water-solubility of 1 mg/ml or more and there is no upper limit of the solubility. The preparation in the present invention can be applied to any drug whose water-solubility is 1 mg/ml or more, accordingly, can also be applied to a drug with water-solubility of about 1 g/ml.

The preparation of the present invention is also applied to a drug with water-solubility of less than 1 mg/ml besides "very soluble" drug and there is no lower limit of the solubility The preparation of the present invention can be applied to any drug with water-solubility of less than 1 mg/ml, accordingly, can be applied to a drug whose water-solubility is about 0.1 ng/ml.

It is preferred for the preparation of the present invention that 50 to 100% of the drug is present in granules, and the remaining exists within the erodible and swelling matrix or the release-modifying layer, or within the matrix and release-modifying layer in directly dispersed form.

The coated swelling-matrix oral preparation for control of drug release, according to the present invention, consists of three components: (1) Granules containing a drug; (2) swelling and erodible matrix where the drug-containing granules are embedded; and (3) a coating layer surrounding the matrix. Considering the drug release mechanism, coating layer provides initial lag-time for a certain amount of time. This is for enteric preparation or for release at specific site in the body. Further, coating layer functions in inhibiting dumping effect of drug release and in raising drug stability under storage. When said controlled-release preparation is exposed in the body fluid, coating layer disappears with swelling of inner matrix after the certain amount of time, leading to active swelling and erosion of the matrix. Swelling and erosion of the matrix leads to controlled-release of granules embedded in matrix and then drug is released in controlled way from the granules. In case of conventional swelling matrix system, direct release of drug from inner matrix leads to tendency of time-dependent decrease of drug release rate, while in case of the system according to the present invention, drug within the granules is directly released into matrix, and at the same time, drug-containing granules are continuously released and drug is released from the granules, i.e. multi-stage controlled-release, accordingly, drug release area increases with time due to cumulated granules to compensate the reduction of release rate according to reduction of surface area of erodible matrix itself, ultimately leading to drug release at constant rate.

The first constitution of the preparation according to the present invention is granules comprising a drug and a carrier material, wherein the size of said granules is 0.1 ~ 1 mm, said carrier material is hydrophobic material in case of drug with water-solubility of 1 mg/ml or more, and hydrophilic material in case of drug with water-solubility of less than 1 mg/ml.

In the preparation of the present invention, it is preferred that in case drug has a water-solubility within range from 1 mg/ml to 100 mg/ml, the drug-containing granules are prepared by wet granulation, and in case the drug has water-solubility of 100 mg/ml or more, the drug-containing granules are prepared into granules by dispersing the drug in hydrophobic fusible materials forming the granules.

Additionally, when water-solubility of the drug is less than 1 mg/ml, it is preferred to prepare the drug-containing granules according to solid dispersion method.

In case of drug with water-solubility of 1 mg/ml or more, it is preferred for said hydrophobic material forming the granules to be at least one selected from the group consisting of fatty acids, fatty acid esters, fatty acid alcohols, fatty acid mono-, di-, tri-glycerides, waxes, hydrogenated castor oil, hydrogenated vegetable oil and as like. Examples of the fatty acid alcohols include cetostearyl alcohol, stearyl alcohol, lauryl alcohol, myristyl alcohol and as like. Examples of the fatty acid esters include glyceryl monostearate, glycerol monooleate, acetylated monoglyceride, tristearin, tripalmitin, cetyl ester wax, glyceryl palmitostearate, glyceryl behanate (Compritol 888 ATO^{™}) and as like. Examples of the waxes include beeswax, carnauba wax, glyco wax, castor wax and as like.

In case of drug with water-solubility of less than 1 mg/ml, for the preparation of the present invention, it is preferable that said hydrophilic carrier material forming granules is at least one selected from the group consisting of polyalkylene glycol and carboxyvinyl hydrophilic polymer. As specific example, polyethyleneglycol with molecular weight of 1,000-6,000, carbomer (Carbopol^{™}), calcium carboxymethylcellulose and sodium carboxymethylcellulose can be enumerated.

The granules of the preparation according to the present invention can further comprise other additives and excipients. As example, lactose, starch, mannitol, saccharose, glucose, sorbitol, dibasic calcium phosphate dihydrate, anhydrous dibasic calcium phosphate, microcrystalline cellulose (Avicel^{™}), gelatin, polyvinylpyrrolidone and salt can be enumerated. The granules can contain at least one of the above additives. The granules can further contain, if necessary, cross-linked sodium carboxymethylcellulose or cross-linked polyvinylpyrrolidone, which accelerates disintegration of granules, and to correct pH dependence of drug, can contain inorganic acid and its conjugate base, or organic acid (such as citric acid and tartaric acid) and its conjugate base. The granules prepared as described above are the part that finally controls release and absorption of drug. In case of hydrophilic drugs, the control is achieved by diffusion through hydrophobic substance forming the granules, while in hydrophobic drugs, hydrophilic substance forming the granules, hydration environment established around the granules and increased surface area improve wettability of drug to increase the water-solubility thereof.

The second constitution of the preparation according to the present invention is matrix having said granule embedded therein, which comprising swelling and erodible polymer(s) and swelling-regulating material(s).

As the swelling and erodible polymer forming the matrix, for the formation of hydrogel matrix, at least one selected from the group consisting of hydroxyalkylcellulose, hydroxypropylakylcellulose, polyalkylene oxide, sodium alginate, povidone, polyvinyl alcohol and sodium carboxymethylcellulose can be used. In particular, it is preferred to use at least one selected from the group consisting of hydroxypropylcellulose, hydroxypropylmethylcellulose, polyethylene oxide, sodium alginate, povidone polyvinyl alcohol and sodium carboxymethyl cellulose.

In addition, the matrix can further include adjuvant for formation of the swelling and erodible matrix, and at least one selected from the group consisting of cross-linked sodium carboxymethylcellulose or cross-linked polyvinylpyrrolidone, lactose, starch, mannitol, saccharose, glucose, sorbitol, dibasic calcium phosphate dihydrate, anhydrous dibasic calcium phosphate, microcrystalline cellulose (Avicel^{™}), gelatin, polyvinylpyrrolidone, magnesium stearate, stearic acid, sodium stearate, talc, sodium benzoate, boric acid and colloidal silica, can be used. Also, the matrix can contain a portion of drug to be contained in granules.

Swelling-regulating material among said matrix components is used to control the degree and velocity of swelling of the polymer, and as the swelling-regulating material, cross-linked sodium carboxymethylcellulose or cross-linked polyvinylpyrrolidone, or a mixture thereof can be used. The swelling-regulating material is preferred to be used in a content of 1 to 10% by weight to the total weight of matrix. The swelling and erodible polymer forming the core matrix provides, via swelling, hydration environment around the granules dispersed within the matrix. In particular, it acts a role of raising drug solubility in case of granules comprising hydrophobic drug. Further, it carries out function, secondary drug release control, by controlling the release of granules from the surface by erosion.

The third constitution of the preparation according to the present invention is release-modifying layer, and comprises at least one selected from the group consisting of hydrophobic release-modifying polymer, hydrophilic release-modifying polymer and pH-dependent release-modifying polymer.

In said release-modifying layer, the term "modifying" means that drug release from the preparation is again controlled by this layer, that is, release-modifying layer.

Hydrophobic release-modifying polymer as adequate material for forming the coating layer includes ethylcellulose, shellac and ammonio methacrylate copolymer (Eudragit RS^{™} or Eudragit RL^{™}) and at least one of them can be used.

As adequate material for forming the coating layer, hydrophilic release-modifying polymer can be selected from the group consisting of hydroxyalkylcellulose and hydoxypropylalkylcellulose and at least one of them can be used, and preferably, selected from the group consisting of hydroxymethylcellulose, hydroxyethylcellulose, hydroxypropylcellulose, hydroxybutylcellulose, hydroxypentylcellulose, hydroxypropylmethylcellulose, hydroxypropylbutylcellulose and hydroxypropylpentylcellulose.

As material suitable for the formation of the coating layer, pH-dependent release-modifying polymer includes generally used enteric polymer. Specifically, it is possible to enumerate as follows: hydroxyalkylcellulose phthalate, hydroxyalkylmethylcellulose phthalate, cellulose acetyl phthalate, sodium cellulose acetate phthalate, cellulose ester phthalate, cellulose ether phthalate and anionic copolymer of methacrylic acid and methyl or ethyl methacrylate. At least one selected from the group consisting of them can be used. As example for the anionic copolymer of methacrylic acid and methyl or ethyl methacrylate, Eudragit L and S can be enumerated.

Said release modifying layer can further includes plasticizer and, for example, it can be selected from the group consisting of castor oil, hydrogenated castor oil, fatty acid, substituted triglycerides and glyceride, polyethylene glycol of molecular weight within range of 300 to 50,000 and its derivatives. Such release modifying layer, i.e. coating layer, acts a role of primary drug release control and functions in modifying zero-order release rate of the matrix core. Using of pH dependent or hydrophobic polymer coating enables target-oriented system. For the coating layer, hydrophobic, hydrophilic and pH dependent polymers are used individually or in a combination of them. Coating solution includes plasticizer in a ratio of 5 to 50% by weight of the coating substance.

It is preferred for said release modifying layer to be 1 to 20% by weight to total weight of matrix. For the preparation of coating solution, water or organic solvent is used and as suitable organic solvent, methanol, ethanol, isopropanol, acetone, chloroform, dichloromethane and a mixture thereof can be used.

The oral drug controlled-release system of the present invention comprises granules containing effective amount of drug, swelling and erodible polymer matrix in which the granules are embedded, and a coating layer surrounding the core matrix consisting of the granules and matrix. It is preferred that granules containing the drug reach 50 to 80% by weight to total weight of the preparation,

In the preparation according to the present invention, examples of the applicable drug is as follows:
therapeutic agents for aconuresis selected from oxybutynin and tolterodine;
calcium channel blockers selected from nifedipine, verapamil, isradipin, nilvadipin, flunarizine, nimodipine, diltiazern, nicardipine, nisoldipin, felodipin, amlodipin, cinarizin and pendilin;
beta-adrenergic antagonists selected from propranolol and metoprolol;
angiotensin-converting enzyme inhibitors selected from captopril, enalapril, ramipril, fosinopril, altiopril, benazepril, libenzapril, alacepril, cilazapril, cilazaprilat, perindopril, zofedopril, lisinopril, imidapril, spirapril, rentiapril, delapril, alindapril, indalapril and quinalapril;
non-steroidal anti-inflammatory agents selected from ketorolac, ketoprofen, benoxaprofen, caprofen, flubiprofen, fenoprofen, suprofen, fenbufen, ibuprofen, indoprofen, naproxen, miroprofen, oxaprozine, pranoprofen, pirprofen, thiaprofenic acid, fluprofen, ahninoprofen, bucloxic acid, alclofenac acematacin, aspirin, indomethacin, ibufenac, isoxepac, profenac, fentiazac, clidanac, oxpinac, sulindac, tolmetin, zomepirac, zidometacin, tenclofenac, tiopinac, mefenamic acid, flufenamic acid, niflumic acid, meclofenamic acid, tolfenamic acid, diflufenisal, isoxicam and sudoxicam:
therapeutic agents for respiratory disorders selected from theophylline, salbutamol, aminophylline, dextromethorphan and pseudoephedrine ;
analgesics selected from tramadol, acetaminophen, morphine, hydromorphone, oxycodone and propoxyphene;
psychoneural drugs selected from fluoxetine, paroxetine, buspirone, bupropion, caxmabazepine, carvidopa, levodopa, methylphenidate, trazodone, valproic acid, amitriptyline, carbamazepine, ergoloid, haloperidol and lorazepam ;
antibiotics selected from azithromycin dihydrate, cepha antibiotics, clarithromycin, doxycycline and nitrofurantonin ;
antihyperlipidemic agent selected from bezafibrate, fenofibrate, ethofibrate and lovastatin;
antidiabetic agent selected from glyburide, glipizide and metformin;
and cyclobenzaprin, favotidin, nizatidine, propafenone, clonazepam, hyoscyamine, diphenhydramine, orlistat and doxazosin.

It is preferable for the granules to be prepared by wet granulation, in case of water-soluble drug. For example, a drug, substance forming the granules as described above and at least one kind of additives are mixed and combined by adding binder solution comprising hydrophilic polymer and water or organic solvent such as denatured anhydrous ethanol as granulating fluid. Granulating fluid is added until wet mixture is formed and then the wet mixture is passed through. 6-18 mesh sieve. This is dried in an oven at 24 to 60°C for 12 to 24 hr. The dried granules are screened with 10-24 mesh sieve.

In case a drug has water-solubility of 50 mg/ml or more, for effective release-delay, drug particles can be covered with hydrophobic substance by melt-granulation. At a temperature of at least melting point of delivery system component, drug and other additives are mixed, dispersed and slowly cooled to obtain solid body of the delivery system, and granules are obtained by pulverization and screening.

In case of hydrophobic drug, it is preferable that drug, granule component described above and at least one additive are admixed, melted at melting point of the granule component to obtain solid dispersion. For example, granule-forming additives are added to the formed solid dispersion until granules are formed. The granules are screened through 6~18 mesh sieve, and then dried in an oven at 24 to 60°C for 12 to 24 hr. The dried granules are screened with 10-24 mesh sieve. Granules prepared as described above are mixed with swelling and erodible polymer and at least one additive forming matrix. Lubricant is added to the mixture and the final mixture is prepared into compressed tablet of core matrix without coating layer. Coating layer is formed by using hydrophobic polymer, hydrophilic polymer and enteric or pH dependent substance, individually or in a mixture. At least one polymer for the formation of coating layer and plasticizer is made ready in a form dispersed in water or organic solvent and then the dispersion solution is sprayed on the core matrix prepared as above. Coated tablet is finally dried in an oven at 40 to 50°C. For stability and color of preparation, seal coating can be conducted. In order to allow drug concentration to rapidly reach effective blood level, 1 to 20% of drug can be directly contained within the coating layer.

Drug release through the multi-stage controlled-release system according to the present invention is controlled via three steps.

At the first step, coating layer, i.e. release-modifying layer exhibits intentional release-delaying effect over a certain amount of time. In case of coating layer consisting of hydrophilic polymer alone, overall release profile is not influenced and release pattern of the core matrix itself is maintained, leading to maintenance of zero-order release profile over an 8 to 24 hr or more periods. In case hydrophilic or enteric polymer is used along with hydrophobic polymer, after release-delay over a certain amount of time is maintained, external fluid is penetrated through pores formed by dissolution of hydrophilic or enteric polymer and hydrophilic plasticizer and the penetrated fluid starts to swell the core matrix. Swelling pressure of the core matrix causes disappearing of coating layer and zero-order release of drug occurs. When coated with enteric polymer, below pH 4.0, there is no release, then at pH 4.0 or more, release starts with loss of the coating layer.

At the second step, swelling of the core matrix actively undergoes upon the disintegration and dissolution of the coating layer, and leads to establishment of hydration environment around the granules embedded in the matrix. As erosion of matrix component starts from the surface of the swelling matrix, granules are to be released by a constant rate.

It is preferred for the preparations of the present invention that, by erosion of the surface of matrix, 0 to 20% of total granules is released over 0 to 4 hr, 0 to 50% is released over 0 to 8 hr, 0 to 70% is released over 0 to 16hr, and 0 to 100% is released over 0 to 24 hr.

At the third step, finally, drug is released by diffusion through pores formed within the granules and by osmotic pressure difference against the external fluid.

Drug release pattern of core matrix itself maintains zero-order release, and introducing of coating layer brings delay over a certain amount of time to lead to intentional appearance of biphasic zero-order release pattern. Release rate can be controlled in various ways by ratio of granules component forming the system and amount of granules, amount of swelling polymer and ratio of swelling matrix to granules, and ratio and amount of hydrophobic, hydrophilic or enteric polymer forming the coating layer.

The system prepared according to the present invention is oral multi-stage controlled-release system and suitable for designing oral drug delivery system taken once or twice a day which exhibits controlled-release for a long time and on specific target for the drug's therapeutic purpose. Drug is released from granules that are released from matrix by swelling and erosion, and cumulated released-granules allow surface area for drug release to be maintained at a constant level. Thus, this compensates the decrease of drug release rate according to reduction of surface area by erosion of matrix, leading to prolonged drug release at constant rate. Maintaining of zero-order release rate enables blood level of drug to be kept at a steady level for a long time.

### Best Mode for Carrying Out the Invention

The Examples given below are just to explain the present invention and, in any case, they should not be regarded as limiting the scope of the present invention, and in view of the detailed description of invention and the patent claims, the Examples and their equivalents are obvious to persons skilled in the art.

### Examples 1~5. Preparations of core matrix tablet containing oxybutynin

Oxybutynin, glyceryl behanate, solubilizer, binder, release-regulating agent and inert diluents were mixed for 10 min at dry state. The mixture, after water was added, was granulated for 5 min. The granules thus formed were screened through 18-mesh sieve and dried in an oven at 24 to 40°C for 12 to 24 hr. The dried granules were screened with 20-mesh sieve. Hydroxypropylmethylcellulose, binders, swelling-regulating agent and diluents were added to the screened granules, and then they were mixed for 10 min. Finally, lubricant was added to them, and then they were mixed for 5 min. The mixture was compressed to prepare tablets. The following Table 1 represents the ingredients of the core matrix tablet.

**Table 1. Compositions of core matrix tablet containing oxybutynin**

| Ingredient (mg) | Example 1 | Example 2 | Example 3 | Example 4 | Example 5 |
|---|---|---|---|---|---|
| Oxybutynin hydrochloride | 5 | 5 | 5 | 5 | 5 |
| Glyceryl behanate | 10 | 10 | 20 | 15 | 15 |
| Dibasic calcium phosphate dihydrate | 35.9 | 45.9 | 55.9 | 56.85 | 28.425 |
| Lactose | - | - | - | - | 28.425 |
| Sodium chloride | - | - | - | 17.63 | 17.63 |
| Sodium lauryl sulfate | 0.1 | 0.1 | 0.1 | 0.15 | 0.15 |
| Povidone | 6 | 6 | 6 | 9 | 9 |
| Cross-linked sodium carboxymethylcellulose | - | - | - | - | 15 |
| Hydroxypropylmethyl cellulose | 40 | 30 | 20 | 45 | 30 |
| Magnesium stearate | 3 | 3 | 3 | 1.5 | 1.5 |
| Total | 100 | 100 | 100 | 150 | 150 |

### Experimental Example 1. Dissolution test for the preparations of Examples 1~5

Release profile of core matrix tablet prepared in said Examples 1-5 was determined by USP dissolution test method under conditions of simulated intestinal fluid (fluid II, pH 6.8), paddle type II and 50 rpm/900 ml and dissolution level according to time was measured. The result was represented by dissolution percentage as function of time in Table 2.

**Table 2. Dissolution percentage (%)**

| Time (hr) | Example 1 | Example 2 | Example 3 | Example 4 | Example 5 |
|---|---|---|---|---|---|
| 0 | 0.00 | 0.00 | 0.00 | 0.00 | 0.00 |
| 1 | 11.03 | 14.47 | 10.51 | 4.78 | 15.27 |
| 2 | 10.74 | 18.56 | 15.51 | 10.29 | 32.75 |
| 3 | 13.53 | 20.30 | 14.81 | 16.01 | 41.93 |
| 4 | 14.18 | 25.22 | 20.77 | 20.00 | 48.53 |
| 6 | 17.07 | 31.54 | 28.14 | 30.65 | 58.80 |
| 8 | 24.04 | 40.52 | 37.91 | 38.86 | 62.73 |
| 10 | 29.81 | 48.68 | 45.35 | 46.23 | 68.64 |
| 12 | 36.70 | 58.42 | 43.76 | 53.48 | 72.06 |
| 24 | 68.74 | 84.54 | 72.98 | 91.73 | 93.01 |

Based on the dissolution test result for the controlled-release preparation of the present invention obtained in Examples 1-5, it was confirmed that various controlled-release patterns of oxybutynin could be obtained by the core matrix tablet itself, and the release rate could be controlled by regulating the content of swelling and erodible polymer and glyceryl behanate. Example 4 represents zero-order release pattern over 24 hr, and Example 5 shows that the release pattern can be affected by the content of swelling-regulating material contained in the matrix.

### Examples 6 and 7. Preparations of core matrix tablet containing oxybutynin

Oxybutynin, glyceryl behanate, solubilizer, binder, release-regulating agent and inert diluents were mixed for 10 min at dry state. The mixture; after water was added, was granulated for 5 min. The granules thus formed were screened through 18-mesh sieve and dried in an oven at 24 to 40°C for 12 to 24 hr. The dried granules were screened with 20-mesh sieve. Polyethylene oxide, binders, swelling-regulating agent and diluents were added to the screened granules, and then they were mixed for 10 min. Finally, lubricant was added to them, and then they were mixed for 5 min. The mixture was compressed to prepare tablets. The following Table 3 represents the ingredients of the core matrix tablet.

**Table 3. Compositions of core matrix tablet containing oxybutynin**

| Ingredient (mg) | Example 6 | Example 7 |
|---|---|---|
| Oxybutynin hydrochloride | 5 | 5 |
| Hydrogenated castor oil | 5 | 15 |
| Dibasic calcium phosphate dihydrate | 65 | 55 |
| Sodium chloride | 17.85 | 17.85 |
| Sodium lauryl sulfate | 0.15 | 0.15 |
| Povidone | 9 | 9 |
| Polyethylene oxide | 45 | 45 |
| Magnesium stearate | 3 | 3 |
| Total | 150 | 150 |

### Experimental Example 2. Dissolution test for the preparations of Examples 6 and 7

Release profiles of the core matrix tablets prepared in said Examples 6 and 7 were determined by USP dissolution test apparatus under conditions of simulated intestinal fluid (fluid II, pH 6.8), paddle type II and 50 rpm/900 ml and dissolution level according to time was measured. The result was represented by dissolution percentage as function of time in Table 4.

**Table 4. Dissolution percentage (%)**

| Time (hr) | Example 6 | Example 7 |
|---|---|---|
| 0 | 0.00 | 0.00 |
| 1 | 5.57 | 3.11 |
| 2 | 10.26 | 4.98 |
| 3 | 10.75 | 6.44 |
| 4 | 15.67 | 8.75 |
| 6 | 24.20 | 14.86 |
| 8 | 60.99 | 49.38 |
| 18 | 67.38 | 59.29 |
| 20 | 67.72 | 62.02 |
| 24 | 71.30 | 66.00 |

### Examples 8-10. Coating of core matrix tablet containing oxybutynin

The core matrix tablet prepared in said Example 2 was coated with a mixture of hydrophilic release-modifying polymer and hydrophobic release-modifying polymer, i.e. hydroxypropylmethylcellulose and ethylcellulose. Coating solution was prepared according to the composition given in Table 5. Spray coating was carried out in pan coater, and then the products were dried in oven at 40 to 50°C for 12 to 24 hr.

**Table 5. Coating Solution Composition**

| Components (%) | Example 8 | Example 9 | Example 10 |
|---|---|---|---|
| Hydroxypropylinethylcellulose | 5.4 | 4.8 | 4.2 |
| Ethylcellulose | 0.6 | 1.2 | 1.8 |
| Castor oil | 0.7 | 0.7 | 0.7 |
| Ethanol | 46.7 | 46.7 | 46.7 |
| Methylene chloride | 46.7 | 46.7 | 46.7 |
| Coating %* | 3 | 3 | 3 |

| | | | |
|---|---|---|---|
| *Coating degree to the weight of uncoated core matrix tablet is represented by %. | | | |

### Experimental Example 3. Dissolution test for the preparations of Examples 8~10

Release profiles of the coated core matrix tablets prepared in said Examples 8-10 were determined by USP dissolution test apparatus under conditions of pH 4.0 solution, paddle type II and 50 rpm/900 ml and time-dependent dissolution level was measured. The result was represented by dissolution percentage as function of time in Table 6.

**Table 6. Dissolution percentage (%)**

| Time (hr) | Example 8 | Example 9 | Example 10 |
|---|---|---|---|
| 0 | 0.00 | 0.00 | 0.00 |
| 1 | 6.16 | 6.07 | 3.74 |
| 2 | 11.53 | 10.67 | 7.07 |
| 3 | 17.28 | 16.01 | 10.59 |
| 4 | 24.66 | 19.82 | 13.69 |
| 6 | 34.47 | 27.63 | 2004 |
| 8 | 45.13 | 34.60 | 27.23 |
| 10 | 54.51 | 41.98 | 31.46 |
| 12 | 63.67 | 50.11 | 37.56 |
| 24 | 100.72 | 85.25 | 69.06 |

The dissolution test results for the coated core matrix of Examples 8 to 10 reveal that drug release rate of core matrix showing zero-order release pattern can be regulated by relative content of hydrophobic release-modifying substance contained in the coating layer.

### Examples 11 and 12. Coating of core matrix tablet containing oxybutynin

The core matrix tablets prepared by said Examples 4 and 5 were coated with a mixture of hydrophobic release-modifying polymer and pore-forming substance, i.e. ethylcellulose and polyethyleneglycol (MW 300). Coating solution was prepared according to the composition given in Table 7. Spray coating was carried out in pan coater, and then the products were dried in oven at 40 to 50°C for 12 to 24 hr.

**Table7. Coating Solution Composition**

| Components (%) | Example 11 | Example 12 |
|---|---|---|
| Ethylcellulose | 7.0 | 7.0 |
| Polyethylene glycol (MW: 300) | 2.8 | 2.8 |
| Ethanol | 90.2 | 90.2 |
| Coating %* | 1.0 | 1.0 |

| | | |
|---|---|---|
| *Degree of coating to the weight of uncoated core matrix tablet is represented by %. | | |

### Experimental Example 4. Dissolution test for the preparations of Examples 11 and 12

Release profiles of the coated core matrix tablet prepared in said Examples 11 and 12 were determined by USP dissolution test apparatus under conditions of simulated intestinal fluid (Fluid II, pH 6.8), paddle type II and 50 rpm/900 ml and time-dependent dissolution level was measured. The result was represented by dissolution percentage as function of time in Table 8.

**Table 8. Dissolution percentage (%)**

| Time (hr) | Example 11 | Example 12 |
|---|---|---|
| 0 | 0.00 | 0.00 |
| 1 | 0.00 | 4.67 |
| 2 | 1.68 | 17.61 |
| 3 | 3.45 | 19.41 |
| 4 | 5.89 | 27.70 |
| 6 | 10.55 | 34.38 |
| 18 | 35.79 | 64.76 |
| 20 | 41.92 | 72.18 |
| 22 | 49.87 | 79.45 |
| 24 | 55.24 | 99.32 |

The dissolution test result for the coated core matrix of Examples 11 and 12 demonstrates that the depth of coating and the content of hydrophilic release-modifying polymer, that is, pore-forming material can modify the drug release rate of core matrix showing zero-order release pattern.

### Examples 13~15. Coated core matrix tablet containing oxybutynin

Preparation process for matrix core is the same as in Examples 1-5. Example 13 includes within granules citric acid, substance for regulating pH-surrounding granules, instead of sodium chloride, and includes swelling-regulating material to control the swelling pressure and the swelling speed of matrix. In case of Examples 14 and 15, swelling-regulating material exists in both granules and matrix. As coating substance, shellac was used, and the compositions of the coating solution and the core matrix are represented in the following Table 9.

**Table 9. Compositions of core matrix tablet containing oxybutynin and coating solution**

| | Ingredient (mg) | Example 13 | Example 14 | Example 15 |
|---|---|---|---|---|
| Core Matrix | Oxybutynin hydrochloride | 5 | 5 | 5 |
| | Glyceryl behanate | 15 | 15 | 15 |
| | Dibasic calcium phosphate dihydrate | 28.425 | 28.425 | 28.425 |
| | Lactose | 31.925 | 41.925 | 41.925 |
| | Sodium chloride | - | 17.35 | 17.35 |
| | Citric acid | 17.5 | - | - |
| | Sodium lauryl sulfate | 0.15 | 0.15 | 0.15 |
| | Povidone | 9 | 9 | 9 |
| | Cross-linked sodium carboxymethylcellulose | 1.5 | 1.65 | 1.65 |
| | Hydroxypropyhnethylcellulose | 30 | 30 | 30 |
| | Magnesium stearate | 1.5 | 1.5 | 1.5 |
| | Moisture* | q.s. | q.s. | q.s. |
| | Total | 150 | 150 | 150 |
| Coating solution | Shellac(OPAGLOSGS-2-0401) | 50% | 50% | 50% |
| | Ethanol | 50% | 50% | 50% |
| | Coating%⁺ | 5 | 1 | 5 |

| | | | | |
|---|---|---|---|---|
| *Removed during treatment process. + Degree of coating to the weight of uncoated core matrix tablet is represented by %. | | | | |

### Experimental Example 5. Dissolution test for the preparations of Examples 13 and 14

Release profiles of the coated core matrix tablets prepared in said Examples 13 and 14 were determined by USP dissolution test apparatus under conditions of simulated intestinal fluid (Fluid II, pH 6.8), paddle type II and 50 rpm/900 ml and time-dependent dissolution level was measured. The result was represented by dissolution percentage as function of time in Table 10.

**Table 10. Dissolution percentage (%)**

| Time (hr) | Example 13 | Example 14 |
|---|---|---|
| 0 | 0.00 | 0.00 |
| 1 | 1.20 | 3.96 |
| 2 | 3.28 | 9.72 |
| 3 | 22.85 | 24.45 |
| 4 | 30.15 | 32.45 |
| 6 | 43.64 | 40.94 |
| 19 | 79.36 | 86.58 |
| 20 | 81.34 | 90.45 |
| 22 | 84.22 | 93.63 |
| 24 | 87.00 | 98.03 |

The dissolution test result for the coated core matrix tablets of Examples 13 and 14 shows that achieving release-delay effect over a certain amount of time by controlling depth of shellac coating leads to biphasic release pattern. The release-delay and the rapid drug release after the period can be induced by regulating the content of swelling-regulating material contained in the core matrix.

### Experimental Example 6. Dissolution test for the preparations of Examples 13~15

Release profiles of the coated core matrix tablets prepared in said Examples 13 to 15 were determined by USP dissolution test method (paddle type II, 50 rpm/900 ml). According to the simulated GI method (Gastrointestinal method), the test was conducted in simulated stomach fluid (Fluid I, pH 1.2) for 2 hr and then under simulated intestinal fluid (Fluid II, pH 6.8), time-dependent dissolution level over 24 hr was measured. The result was represented by dissolution percentage as function of time in Table 11.

**Table 11. Dissolution percentage (%)**

| Time (hr) | Example 13 | Example 14 | Example 15 |
|---|---|---|---|
| 0 | 0.00 | 0.00 | 0.00 |
| 0.5 | 1.97 | 10.29 | 4.78 |
| 1 | 7.02 | 24.50 | 10.03 |
| 1.5 | 15.34 | 33.90 | 20.96 |
| 2 | 20.54 | 44.03 | 28.13 |
| 3 | 28.87 | 51.67 | 41.58 |
| 4 | 35.30 | 55.25 | 40.00 |
| 6 | 46.86 | 62.19 | 47.18 |
| 18 | 73.23 | 89.89 | 85.36 |
| 20 | 76.85 | 92.43 | 85.02 |
| 22 | 81.44 | 94.67 | 86.37 |
| 24 | 83.50 | 96.41 | 91.26 |

### Examples 16-18. Coated core matrix tablet containing oxybutynin

Preparation process of matrix core is the same as in Examples 1-5. Example 16 includes swelling-regulating material within granules and matrix to control swelling pressure and swelling speed of matrix. In case of Examples 17 and 18, the content of swelling and erodible polymer within the matrix was increased or reduced, respectively. As coating substance, a mixture of 1:1 ratio of enteric polymer, i. e. hydroxypropylmethylcellulose phthalate, and shellac was used. Compositions of the coating solution and core matrix are represented in Table 12.

**Table 12. Compositions of core matrix tablet containing oxybutynin and coating solution**

| | Ingredient (mg) | Example 16 | Example 17 | Example 18 |
|---|---|---|---|---|
| Core Matrix | Oxybutynin hydrochloride | 5 | 5 | 5 |
| | Glyceryl behanate | 15 | 15 | 15 |
| | Dibasic calcium phosphate dihydrate | 28.425 | 28.425 | 28.425 |
| | Lactose | 41.925 | 41.925 | 26.925 |
| | Sodium chloride | 17.35 | 17.35 | 17.35 |
| | Citric acid | - | - | - |
| | Sodium lauryl sulfate | 0.15 | 0.15 | 0.15 |
| | Povidone | 9 | 16.5 | 9 |
| | Cross-linked sodium carboxymethylcellulose | 1.65 | 1.65 | 1.65 1.03 |
| | Hydroxypropylmethyl cellulose | 30 | 22.5 | 45 |
| | Magnesium stearate | 1.5 | 1.5 | 1.5 |
| | Moisture* | q.s. | q.s. | q.s. |
| | Total | 150 mg | 150 mg | 150 mg |
| Coating solution | Shellac (OPAGLOS GS-2-0401) | 2.68% | 2.68% | 2.68% |
| | Hydroxypropylmethyl cellulose phthalate | 2.68% | 2.68% | 2.68% |
| | Methylene chloride | 48.66% | 48.66% | 48.66% |
| | Ethanol | 45.99% | 45.99% | 45.99% |
| | Coating%⁺ | 4 | 4 | 4 |

| | | | | |
|---|---|---|---|---|
| *Removed during treatment process. + Degree of coating to the weight of the uncoated core matrix tablet is represented by %. | | | | |

### Experimental Example 7. Dissolution test for the preparations of Examples 16~18

Release profiles of the coated core matrix tablets prepared in said Examples 16 to 18 were determined by USP dissolution test method (paddle type II, 50 rpm/900 ml), and according to the simulated GI method (Gastrointestinal method). The test was conducted in simulated stomach fluid (Fluid I, pH 1.2) for 2 hr and then under simulated intestinal fluid (Fluid II, pH 6.8), time-dependent dissolution level over 24 hr was measured. The result was represented by dissolution percentage as function of time in Table 13.

**Table 13. Dissolution percentage (%)**

| Time (hr) | Example 16 | Example 17 | Example 18 |
|---|---|---|---|
| 0 | 0.00 | 0.00 | 0.00 |
| 0.5 | 0.00 | 0.00 | 0.00 |
| 1 | 0.00 | 0.00 | 0.00 |
| 1.5 | 0.00 | 0.00 | 0.00 |
| 2 | 0.00 | 0.00 | 0.00 |
| 3 | 5.01 | 0.00 | 0.00 |
| 4 | 8.55 | 2.29 | 3.31 |
| 6 | 18.51 | 14.52 | 11.09 |
| 8 | 28.50 | 32.33 | 19.86 |
| 18 | 73.27 | 77.65 | 51.32 |
| 20 | 75.66 | 82.15 | 55.05 |
| 22 | 78.63 | 81.52 | 55.15 |
| 24 | 81.87 | 83.72 | 58.58 |

The dissolution test result for the coated core matrix of Examples 14 to 16 represents that pH-dependent release of drug could be corrected by introducing substance with pH dependency into the coating layer, and that drug release was inhibited during the stay in stomach for 2-3 hr and, thereafter, exhibited zero-order release pattern up to 24 hr.

### Example 19. Coated core matrix tablet containing Ketorolac

Ketorolac tromethamine, glyceryl behanate, solubilizer, binder, release-regulating material and inert diluents were mixed for 10 min at dry state. The mixture, after water was added, was granulated for 5 min. The granules thus formed were screened through 18-mesh sieve and dried in an oven at 24 to 40°C for 12 to 24 hr. The dried granules were screened with 20-mesh sieve. Hydroxypropylmethyl cellulose, binders, swelling-regulating agent and diluents were added to the screened granules, and then they were mixed for 10 min. Finally, lubricant was added to them, and then they were mixed for 5 min. The mixture was compressed to prepare tablets. Thus prepared core matrix tablets were spray coated in pan coater and dried in oven at 40 to 50°C for 12 to 24 hr. The following Table 14 represents the ingredients of the core matrix tablet and composition of the coating solution.

**Table 14. Composition of the core matrix tablet and the coating solution**

| | Ingredient (mg) | Example 19 |
|---|---|---|
| Core Matrix | Ketorolac tromethamine | 10 |
| | Glyceryl behanate | 30 |
| | Dibasic calcium phosphate dihydrate | 39.35 |
| | Sodium chloride | 15 |
| | Sodium lauryl sulfate | 0.15 |
| | Povidone | 9 |
| | Hydroxypropylmethylcellulose | 45 |
| | Magnesium stearate | 1.5 |
| | Moisture* | q.s. |
| | Total | 150 |
| Coating solution | Hydroxypropylmethylcellulose | 9.6% |
| | Ethyl cellulose | 2.4% |
| | Methylene chloride | 93.4% |
| | Ethanol | 93.4% |
| | Castor oil | 1.2% |
| | Coating%⁺ | 10 |

| | | |
|---|---|---|
| *Removed during treatment process. + Degree of coating to the weight of the uncoated core matrix tablet is represented by %. | | |

### Experimental Example 8. Dissolution test for the preparations of Example 19

Release profile of the coated core matrix tablet prepared in said Example 17 was determined by USP dissolution test method under condition of simulated intestinal fluid (Fluid II, pH 6.8), paddle type II and 50 rpm/900 ml, and time-dependent dissolution level was measured. The result was represented by dissolution percentage as function of time in Table 15.

**Table 15. Dissolution percentage (%)**

| Time (hr) | Example 19 |
|---|---|
| 0 | 0.00 |
| 1 | 20.61 |
| 2 | 33.43 |
| 3 | 44.80 |
| 4 | 54.33 |
| 6 | 70.26 |
| 8 | 83.40 |
| 12 | 96.17 |

Ketorolac was released from the coated core matrix tablets of Example 19 at a constant rate up to 12 hr, and the release rate could be regulated by the content of swelling material within the matrix and by the coating depth.

### Example 20. Coated core matrix tablet containing enalapril maleate

Therapeutic composition containing enalapril maleate according to the present invention is prepared as follows. First, enalapril maleate, glyceryl behanate, solubilizer, binder, release-regulating substance and inert diluents were mixed for 10 min at dry state. The mixture, after water was added, was granulated for 5 min. Granules thus formed was screened through 18-mesh sieve and dried in an oven at 24 to 40°C for 12 to 24 hr. The dried granules were screened with 20-mesh sieve. Hydroxypropylmethylcellulose, binders, swelling-regulating agent and diluents were added to the screened granules, and then they were mixed for 10 min. Finally, magnesium stearate was added to them, and then they were mixed for 5 min. The mixture was compressed to prepare tablets. Thus prepared core matrix tablets were spray coated in pan coater and dried in oven at 40 to 50°C for 12 to 24 hr. The following Table 16 represents the ingredients of the core matrix tablet and composition of the coating solution.

**Table 16. Compositions of core matrix tablet and coating solution**

| | Ingredient (mg) | Example 20 |
|---|---|---|
| Core Matrix | Enalapril maleate | 10 |
| | Glyceryl behanate | 30 |
| | Dibasic calcium phosphate dihydrate | 39.35 |
| | Sodium chloride | 15 |
| | Sodium lauryl sulfate | 0.15 |
| | Povidone | 9 |
| | Hydroxypropylmethylcellulose | 45 |
| | Magnesium stearate | 1.5 |
| | Moisture* | q.s. |
| | Total | 150 |
| Coating solution | Hydroxypropylmethylcellulose | 9.6% |
| | Ethyl cellulose | 2.4% |
| | Methylene chloride | 93.4% |
| | Ethanol | 93.4% |
| | Castor oil | 1.2% |
| | Coating%⁺ | 10 |

| | | |
|---|---|---|
| *Removed during treatment process +Degree of coating to the weight of the uncoated core matrix tablet is represented by %. | | |

### Experimental Example 9. Dissolution test for the preparations of Example 18

Release profile of the coated core matrix tablet prepared in said Example 18 was determined by USP dissolution test method under conditions of simulated intestinal fluid (Fluid II, pH 6.8), paddle type II and 50 rpm/900 ml, and time-dependent dissolution level was measured. The result was represented by dissolution percentage as function of time in Table 17.

**Table 17. Dissolution percentage (%)**

| Time (hr) | Example 18 |
|---|---|
| 0 | 0.00 |
| 1 | 20.61 |
| 2 | 33.43 |
| 3 | 44.80 |
| 4 | 54.33 |
| 6 | 70.26 |
| 8 | 83.40 |
| 12 | 96.17 |

### Example 21. Coated core matrix tablet containing captopril

Therapeutic composition containing captopril according to the present invention is prepared as follows. First, captopril, glyceryl behanate, solubilizer, binder, release-regulating substance and inert diluents were mixed for 10 min at dry state. The mixture, after water was added, was granulated for 5 min. Granules thus formed was screened through 18-mesh sieve and dried in an oven at 24 to 40°C for 12 to 24 hr. The dried granules were screened with 20-mesh sieve. Hydroxypropylmethylcellulose, binders, swelling-regulating agent and diluents were added to the screened granules, and then they were mixed for 10 min. Finally, magnesium stearate was added to them, and then they were mixed for 5 min. The mixture was compressed to prepare tablets. Thus prepared core matrix tablets were spray coated in pan coater and dried in oven at 40 to 50°C for 12 to 24 hr. Ingredients of the core matrix tablet and composition of the coating solution are shown in Table 18.

**Table 18. Compositions of core matrix tablet and coating solution**

| | Ingredient (mg) | Example 21 |
|---|---|---|
| Core Matrix | Captopril | 25 |
| | Glyceryl behanate | 62.5 |
| | Dibasic calcium phosphate dihydrate | 5 |
| | Povidone | 5 |
| | Hydroxypropylmethylcellulose | 150 |
| | Magnesium stearate | 2.5 |
| | Moisture* | q.s. |
| | Total | 250 |
| Coating solution | Hydroxypropylmethylcellulose | 9.6% |
| | Ethyl cellulose | 2.4% |
| | Methylene chloride | 93.4% |
| | Ethanol | 93.4% |
| | Castor oil | 1.2% |
| | Coating%⁺ | 10 |

| | | |
|---|---|---|
| *Removed during treatment process +Degree of coating to the weight of the uncoated core matrix tablet is represented by %. | | |

### Experimental Example 10. Dissolution test for the preparations of Example 21

Release profile of the coated core matrix tablet prepared in said Example 19 was determined by USP dissolution test method under conditions of simulated intestinal fluid (Fluid II, pH 6.8), paddle type II and 50 rpm/900 ml, and time-dependent dissolution level was measured. The result was represented by dissolution percentage as function of time in Table 19.

**Table 19. Dissolution percentage (%)**

| Time (hr) | Example 21 |
|---|---|
| 0 | 0.00 |
| 1 | 13.64 |
| 2 | 23.51 |
| 3 | 33.40 |
| 4 | 38.77 |
| 8 | 61.48 |
| 19 | 80.67 |
| 20 | 82.13 |
| 22 | 84.19 |
| 24 | 90.79 |

### Example 22. Preparation of core matrix tablets containing diltiazem

Therapeutic composition containing diltiazem according to the present invention is prepared as follows. First, diltiazem hydrochloride, glyceryl behanate, solubilizer, binder, release-regulating substance and inert diluents were mixed for 10 min at dry state. The mixture, after water was added, was granulated for 5 min. Granules thus formed was screened through 18-mesh sieve and dried in an oven at 24 to 40°C for 12 to 24 hr. The dried granules were screened with 20-mesh sieve. Hydroxypropylmethylcellulose, binders, swelling-regulating agent and diluents were added to the screened granules, and then they were mixed for 10 min. Finally, magnesium stearate was added to them, and then they were mixed for 5 min. The mixture was compressed to prepare tablets. Ingredients of the core matrix tablet are shown in Table 20.

**Table 20. Compositions of core matrix tablet containing diltiazem**

| | Ingredient (mg) | Example 22 |
|---|---|---|
| Core Matrix | Diltiazem hydrochloride | 90 |
| | Glyceryl behanate | 40 |
| | Dibasic calcium phosphate dihydrate | 90 |
| | Sodium chloride | 45 |
| | Sodium lauryl sulfate | 1 |
| | Povidone | 10 |
| | Hydroxypropylmethylcellulose | 120 |
| | Magnesium stearate | 4 |
| | Moisture* | q.s. |
| | Total | 400 |

| | | |
|---|---|---|
| *Removed during treatment process | | |

### Experimental Example 11. Dissolution test for the preparations of Example 22

Release profile of the coated core matrix tablet prepared in said Example 22 was determined by USP dissolution test method under conditions of simulated intestinal fluid (Fluid II, pH 6.8), paddle type II and 50 rpm/900 ml, and time-dependent dissolution level was measured. The result was represented by dissolution percentage as function of time in Table 21.

**Table 21. Dissolution percentage (%)**

| Time (hr) | Example 22 |
|---|---|
| 0 | 0.00 |
| 1 | 13.40 |
| 2 | 20.94 |
| 3 | 27.56 |
| 4 | 33.58 |
| 6 | 45.12 |
| 8 | 55.18 |
| 10 | 64.38 |
| 12 | 72.01 |
| 16 | 90.50 |
| 20 | 100.72 |

### Example 23. Preparation of core matrix tablets containing theophylline

Therapeutic composition containing theophylline according to the present invention is prepared as follows. First, theophylline hydrochloride, glyceryl behanate, solubilizer, binder, release-regulating substance and inert diluents were mixed for 10 min at dry state. The mixture, after water was added, was granulated for 5 min. Granules thus formed was screened through 18-mesh sieve and dried in an oven at 24 to 40°C for 12 to 24 hr. The dried granules were screened with 20-mesh sieve. Hydroxypropylmethylcellulose, binders, swelling-regulating agent and diluents were added to the screened granules, and then they were mixed for 10 min. Finally, magnesium stearate was added to them, and then they were mixed for 5 min. The mixture was compressed to prepare tablets. Ingredients of the core matrix tablet are shown in Table 22.

**Table 22. Composition of core matrix tablet containing theophylline**

| | Ingredient (mg) | Example 23 |
|---|---|---|
| Core Matrix | Theophylline | 200 |
| | Glyceryl behanate | 80 |
| | Dibasic calcium phosphate dihydrate | 380 |
| | Sodium chloride | 90 |
| | Sodium lauryl sulfate | 2 |
| | Povidone | 20 |
| | Hydroxypropylmethylcellulose | 120 |
| | Magnesium stearate | 8 |
| | Moisture* | q.s. |
| | Total | 900 |

| | | |
|---|---|---|
| *Removed during treatment process | | |

### Experimental Example 12. Dissolution test for the preparations of Example 23

Release profile of the coated core matrix tablet prepared in said Example 23 was determined by USP dissolution test method under conditions of simulated intestinal fluid (Fluid II, pH 6.8), paddle type II and 50 rpm/900 ml, and time-dependent dissolution level was measured. The result was represented by dissolution percentage as function of time in Table 23.

**Table 23. Dissolution percentage (%)**

| Time (hr) | Example 23 |
|---|---|
| 0 | 0.00 |
| 1 | 11.83 |
| 2 | 17.60 |
| 3 | 22.65 |
| 4 | 26.87 |
| 6 | 35.11 |
| 8 | 41.73 |
| 10 | 47.61 |
| 12 | 50.37 |
| 24 | 72.19 |

The present invention can provide a constant release rate over an 8 to 24 hr or more period by allowing drug to be released from granules released from matrix, as well as directly from inside of the matrix, and by regulating the release rate of the granules by the content of swelling-regulating material within the matrix. Further, the present invention minimized solubility-limit of drug by applying a suitable manufacturing method and components of the granules in consideration of water-solubility of drug.

### Industrial Applicability

The present invention provides oral drug controlled-release preparation with sustained-release effect proper to the characteristics of drug action, as well as with improved stability, by inducing zero-order release through effectively allowing drug release area to be maintained at a fixed level and through introducing a release-modifying layer.

## Claims

1. A controlled-release oral preparation **characterized in that** release of granules from matrix and drug release from the granules are conducted in stepwise way, wherein the preparation comprises:
(1) granules comprising a drug and a carrier material in size of 0.1-1 mm, said carrier material is hydrophobic material in case of drug with water-solubility of 1 or more and said carrier material is hydrophilic material in case of drug with water-solubility of less than 1 mg/ml ;
(2) a matrix in which said granules are embedded, comprising swelling and erodible polymer and swelling-regulating material; and
(3) a release-modifying layer comprising hydrophobic release-modifying polymer, hydrophilic release-modifying polymer, pH-dependent release-modifying polymer or a mixture thereof.

2. The controlled-release oral preparation in Claim 1, wherein 50 to 100% of the drug is present within the granules, and the remaining drug exists within the matrix or the release-modifying layer, or within the matrix and the release-modifying layer in directly dispersed form.

3. The controlled-release oral preparation in Claim 1, wherein the drug has a water-solubility within range from 1 mg/ml to 100 and the granules containing the drug is prepared by wet granulation.

4. The controlled-release oral preparation in Claim 1, wherein the drug has a water-solubility of at least 100 mg/ml, and the granules containing the drug is prepared in granular form by dispersing the drug in fusion of granules components.

5. The controlled-release oral preparation in Claim 1, wherein the drug has a water-solubility of less than 1 mg/ml, and the granules containing the drug is prepared by solid dispersion, method.

6. The controlled-release oral preparation in Claim 1, wherein the hydrophobic material is at least one selected from the group consisting of fatty acids, fatty acid esters, fatty acid alcohols, fatty acid mono-, di-, tri-glycerides, waxes, hydrogenated castor oil and hydrogenated vegetable oil.

7. The controlled-release oral preparation in Claim 6, wherein the fatty acid alcohol is at least one selected from the group consisting of cetostearyl alcohol, stearyl alcohol, lauryl alcohol and myristyl alcohol; fatty acid ester is at least one selected from the group consisting of glyceryl monostearate, glycerol monooleate, acetylated monoglyceride, tristearin, tripalmitin, cetyl ester wax, glyceryl palmitostearate and glyceryl behanate; and wax is at least one selected from the group consisting of beeswax, carnauba wax, glyco wax and castor wax.

8. The controlled-release oral preparation in Claim 1, wherein the hydrophilic material is at least one selected from the group consisting of polyalkylene glycol and carboxyvinyl hydrophilic polymer, and the drug is solid-dispersed in said hydrophilic polymer.

9. The controlled-release oral preparation in Claim 1, wherein the swelling and erodible polymer is at least one selected from the group consisting of hydroxypropyl cellulose, hydroxypropylmethylcellulose, polyethylene oxide, sodium alginate, povidone, polyvinyl alcohol and sodium carboxymethylcellulose.

10. The controlled-release orale preparation in Claim 1, wherein said swelling-regulating material is at least one selected from the group consisting of cross-linked sodium carboxymethylcellulose and cross-linked polyvinylpyrrolidone.

11. The controlled-release oral preparation in Claim 1, wherein said hydrophobic release-modifying polymer used for the formation of release-modifying layer, is at least one selected from the group consisting of ethylcellulose, shellac and ammonio methacrylate copolymer; said hydrophilic release-modifying polymer is at least one selected from the group consisting of hydroxyalkylcellulose and hydroxypropylalkylcellulose ; and said pH-dependent release-modifying polymer is at. least one selected from the group consisting of hydroxyalkylcellulose phthalate, hydroxyalkylmethylcellulose phthalate, cellulose acetyl phthalate, sodium cellulose acetate phthalate, cellulose ester phthalate, cellulose ether phthalate, and anionic copolymer of methacrylic acid with methyl or ethyl methacrylate.

12. The controlled-release oral preparation in Claim 1, wherein said release-modifying layer is 1 to 20% by weight to total weight of matrix, and the granules containing the drug reach 50 to 80%, by weight to total weight of the preparation.

13. The controlled-release oral preparation in Claim 1, wherein the drug is selected from the following group:
therapeutic agents for aconuresis, selected from oxybutynin and tolterodine;
calcium channel backers selected from nifedipine, verapamil, isradipin, nilvadipin, flunarizine, nimodipme, diltiazem, nicardipine, nisoldipin, felodipin, amlodipin, cinarizin and pendilin;
beta-adrenergic antagonists selected from propranolol and metoprolol;
angiotensin-converting enzyme inhibitors selected from captopril, enalaplil, ramipril, fosinopril, altiopril, benazepril, libenzapril, alacepril, cilazapril, citazaprilat, perindopril, zofedopril, lisinopril, imidapril, spirapril, rentiapril, delapril, alindapril, indalapril and quinalapril;
non-steroidal anti-inflammatory agents selected from ketorolac, ketoprofen, benoxaprofen, caprofen, flubiprofen, fenoprofen, suprofen, fenbufen, ibuprofen, indoprofen, naproxen, miroprofen, oxaprozine, pranoprofen, pirprofen, thiaprofenic acid, fluprofen, alminoprofen, bucloxic acid, alclofenac acematacin, aspirin, indomethacin, ibufenac, isoxepac, profenac, fentiazac, clidanac, oxpinac, sulindac, tolmetin, zomepirac, zidometacin, tenclofenac, tiopinac, mefenamic acid, flurenamic acid, niflumic acid, meclofenamic acid, tolfenamic acid, diflufenisal, isoxicam and sudoxicam;
therapeutic agents for respiratory disorders, selected from theophylline, salbutamol, aminophylline, dextromethorphan and pseudoephedrine ;
analgesics selected from tramadol, acetaminophen, morphine, hydromorphone, oxycodone and propoxyphene;
psychoneural drugs selected from fluoxetine, paroxetine, buspirone, carmabazepine, carvidopa, levodopa, methylphenidate, trazodone, valproic acid, amitriptyline, carbamazepine, ergoloid, haloperidol and lorazepam;
antibiotics selected from azithromycin dihydrate, cepha antibiotics, clarithromycin, doxycycline and nitrofurantonin;
antihyperlipidernic agents selected from bezafibrate, fenofibrate, ethofibrate and lovastatin;
antidiabetic agents selected from glyburide, glipizide and metformin; and
cyclobenzaprin, favotidin, nizatidine, propafenone, clonazopam, hyoscyamine, diphenhydramine, orlistat and doxazosin.

14. The controlled-release oral preparation in Claim 1, wherein the drug is released in zero-order over at least 8 to 24 hr upon the administration of the preparation.

15. The controlled-release oral preparation in Claim 1, wherein by erosion of the surface of matrix, 0 to 20% of total granules is released over 0 to 4 hr, 0 to 50% is released over 0 to 8 hr, 0 to 70% is released over 0 to 16hr, and 0 to 100% is released over 0 to 24 hr.

## Patentansprüche

1. Oralpräparat mit kontrollierter Freisetzung, **dadurch gekennzeichnet, dass** die Freisetzung von Granulat aus der Matrix und die Freisetzung von Arzneimittel aus dem Granulat schrittweise erfolgen, wobei das Präparat umfasst:
(1) Granulat umfassend ein Arzneimittel und ein Trägermaterial in der Größe von 0,1-1 mm, wobei es sich bei dem Trägermaterial um ein hydrophobes Material handelt, falls das Arzneimittel eine Löslichkeit in Wasser von 1 mg/ml oder mehr aufweist, und es sich bei dem Trägermaterial um ein hydrophiles Material handelt, falls das Arzneimittel eine Löslichkeit in Wasser von weniger als 1 mg/ml aufweist,
(2) eine Matrix, in der das Granulat eingebettet ist, umfassend quellfähiges und erodierbares Polymer und Aufquellen-regulierendes Material, und
(3) eine Freisetzungs-modifizierende Schicht umfassend hydropohobes Freisetzungs-modifizierendes Polymer, hydrophiles Freisetzungs-modifizierendes Polymer, pH-abhängiges Freisetzungs-modifizierendes Polymer oder eine Mischung derselben.

2. Oralpräparat mit kontrollierter Freisetzung nach Anspruch 1, wobei 50 bis 100% des Arzneimittels im Granulat vorhanden ist und das restliche Arzneimittel in der Matrix oder der Freisetzungs-modifizierenden Schicht vorhanden ist oder in direkt dispergierter Form in der Matrix und der Freisetzungs-modifizierenden Schicht vorhanden ist.

3. Oralpräparat mit kontrollierter Freisetzung nach Anspruch 1, wobei das Arzneimittel eine Löslichkeit in Wasser im Bereich von 1 mg/ml bis 100 mg/ml aufweist und das Granulat, welches das Arzneimittel enthält, durch Nassgranulierung zubereitet wird.

4. Oralpräparat mit kontrollierter Freisetzung nach Anspruch 1, wobei das Arzneimittel eine Löslichkeit in Wasser von mindestens 100 mg/ml aufweist und das Granulat, welches das Arzneimittel enthält, in körniger Form zubereitet wird, indem das Arzneimittel in einer Schmelze aus Granulatkomponenten dispergiert wird.

5. Oralpräparat mit kontrollierter Freisetzung nach Anspruch 1, wobei das Arzneimittel eine Löslichkeit in Wasser von weniger als 1 mg/ml aufweist und das Granulat, welches das Arzneimittel enthält, durch ein Feststoffdispersionsverfahren zubereitet wird.

6. Oralpräparat mit kontrollierter Freisetzung nach Anspruch 1, wobei es sich bei dem hydrophoben Material um mindestens eines handelt, das aus der aus Fettsäuren, Fettsäureestern, Fettsäurealkoholen, Fettsäure-Mono-, -Di-, -Triglyceriden, Wachsen, hydriertem Rizinusöl und hydriertem Pflanzenöl bestehenden Gruppe ausgewählt ist.

7. Oralpräparat mit kontrollierter Freisetzung nach Anspruch 6, wobei es sich bei dem Fettsäurealkohol um mindestens einen handelt, das aus der aus Cetostearylalkohol, Stearylalkohol, Laurylalkohol und Myristylalkohol bestehenden Gruppe ausgewählt ist, es sich bei dem Fettsäurester um mindestens einen handelt, der aus der aus Glycerylmonostearat, Glycerinmonooleat, acetyliertem Monoglycerid, Tristearin, Tripalmitin, Cetylesterwachs, Glycerylpalmitostearat und Glycerylbehanat bestehenden Gruppe ausgewählt ist, und es sich bei dem Wachs um mindestens eines handelt, das aus der aus Bienenwachs, Carnaubawachs, Glycowachs und Rizinuswachs bestehenden Gruppe ausgewählt ist.

8. Oralpräparat mit kontrollierter Freisetzung nach Anspruch 1, wobei es sich bei dem hydrophilen Material um mindestens eines handelt, das aus der aus Polyalkylenglycol und hydrophilem Carboxyvinyl-Polymer bestehenden Gruppe ausgewählt ist, und das Arzneimittel in dem hydrophilen Polymer Feststoff-dispergiert ist.

9. Oralpräparat mit kontrollierter Freisetzung nach Anspruch 1, wobei es sich bei dem quellfähigen und erodierbaren Polymer um mindestens einen handelt, der aus der aus Hydroxypropylcellulose, Hydroxypropylmethylcellulose, Polyethylenoxid, Natriumalginat, Povidon, Polyvinylalkohol und Natriumcarboxymethylcellulose bestehenden Gruppe ausgewählt ist.

10. Oralpräparat mit kontrollierter Freisetzung nach Anspruch 1, wobei es sich bei dem Aufquell-regulierenden Material um mindestens eines handelt, das aus der aus vernetzter Natriumcarboxymethylcellulose und vernetztem Poyvinylpyrrolidon bestehenden Gruppe ausgewählt ist.

11. Oralpräparat mit kontrollierter Freisetzung nach Anspruch 1, wobei es sich bei dem zur Bildung der Freisetzungs-modifizierenden Schicht verwendeten Freisetzungs-modifizierenden Polymer um mindestens eines handelt, das aus der aus Ethylcellulose, Schellack und Ammonium-methacrylat-Copolymer bestehenden Gruppe ausgewählt ist, es sich bei dem hydrophilen Freisetzungs-modifizierenden Polymer um mindestens eines handelt, das aus der aus Hydroxyalkylcellulose und Hydroxypropylalkylcellulose bestehenden Gruppe ausgewählt ist, und es sich bei dem pH-abhängigen Freisetzungs-modifizierenden Polymer um mindestens eines handelt, das aus der aus Hydroxyalkylcellulosephthalat, Hydroxyalkylmethylcellulosephthalat, Celluloseacetylphthalat, Natriumcelluloseacetatphthalat, Celluloseesterphthalat, Celluloseetherphthalat und anionisches Copolymer aus Methacrylsäure und Methyl- oder Ethylmethacrylat bestehenden Gruppe ausgewählt ist.

12. Oralpräparat mit kontrollierter Freisetzung nach Anspruch 1, wobei die Freisetzungs-modifizierende Schicht 1 bis 20 Gewichts-% des Gesamtgewichts der Matrix ausmacht und das Granulat, welches das Arzneimittel enthält, 50 bis 80 Gewichts-% des Gesamtgewichts des Präparats erreicht.

13. Oralpräparat mit kontrollierter Freisetzung nach Anspruch 1, wobei das Arzneimittel aus der folgenden Gruppe ausgewählt ist:
Therapeutika für Harninkontinenz, ausgewählt aus Oxybutynin und Tolterodin,
Kalziumkanalblocker ausgewählt aus Nifedipin, Verapamil, Isradipin, Nilvadipin, Flunarizin, Nimodipin, Diltiazem, Nicardipin, Nisoldipin, Felodipin, Amlodipin, Cinarizin und
Pendilin,
Beta-adrenerge Antagonisten ausgewählt aus Propranolol und
Metoprolol,
ACE-Hemmer (Hemmstoffe des Angiotensin-converting Enzyms) ausgewählt aus Captopril, Enalapril, Ramipril, Fosinopril, Altiopril, Benazepril, Libenzapril, Alacepril, Cilazapril, Cilazaprilat, Perindopril, Zofedopril, Lisinopril, Imidapril, Spirapril, Rentiapril, Delapril, Alindapril, Indalapril und
Quinalapril,
nicht-steroidale Antirheumatika ausgewählt aus Ketorolac, Ketoprofen, Benoxaprofen, Caprofen, Flubiprofen, Fenoprofen, Suprofen, Fenbufen, Ibuprofen, Indoprofen, Naproxen, Miroprofen, Oxaprozin, Pranoprofen, Pirprofen, Thiaprofensäure, Fluprofen, Alminoprofen, Bucloxinsäure,
Alclofenac Acematacin, Aspirin, Indomethacin, Ibufenac, Isoxepac, Profenac, Fentiazac, Clidanac, Oxpinac, Sulindac, Tolmetin, Zomepirac, Zidometacin, Tenclofenac, Tiopinac, Mefenaminsäure, Flufenaminsäure, Nifluminsäure, Meclofenaminsäure, Tolfenaminsäure, Diflufenisal, Isoxicam und
Sudoxicam,
Therapeutika für Atemwegsstörungen ausgewählt aus Theophyllin, Salbutamol, Aminophyllin, Dextromethorphan und Pseudoephedrin,
Analgetika ausgewählt aus Tramadol, Acetaminophen, Morphin, Hydromorphon, Oxycodon und Propoxyphen,
psychoneurale Arzneimittel ausgewählt aus Fluoxetin, Paroxetin, Buspiron, Carmabazepin, Carvidopa, Levodopa,
Methylphenidat, Trazodon, Valproinsäure, Amitriptylin, Carbamazepin, Ergoloid, Haloperidol und Lorazepam,
Antibiotika ausgewählt aus Azithromycindihydrat, Cepha-Antibiotika, Clarithromycin, Doxycyclin und Nitrofurantonin, Hyperlipidämiemittel ausgewählt aus Bezafibrat, Fenofibrat, Ethofibrat und Lovastatin,
Diabetesmittel ausgewählt aus Glyburid, Glipizid und Metformin, und
Cyclobenzaprin, Favotidin, Nizatidin, Propafenon, Clonazepam, Hyoscyamin, Diphenhydramin, Orlistat und Doxazosin.

14. Oralpräparat mit kontrollierter Freisetzung nach Anspruch 1, wobei das Arzneimittel nach seiner Verabreichung mit einer Kinetik nullter Ordnung über eine Dauer von mindestens 8 bis 24 Stunden freigesetzt wird.

15. Oralpräparat mit kontrollierter Freisetzung nach Anspruch 1, wobei durch Erosion der Matrixoberfläche 0 bis 20% des gesamten Granulats über eine Dauer von 0 bis 4 Stunden freigesetzt werden, 0 bis 50% des gesamten Granulats über eine Dauer von 0 bis 8 Stunden freigesetzt werden, 0 bis 70% des gesamten Granulats über eine Dauer von 0 bis 16 Stunden freigesetzt werden und 0 bis 100% des gesamten Granulats über eine Dauer von 0 bis 24 Stunden freigesetzt werden.

## Revendications

1. Préparation orale à libération contrôlée, **caractérisée en ce que** la libération de granules par une matrice et la libération d'un médicament par les granules sont effectuées par étapes, la préparation comprenant :
(1) des granules comportant un médicament et un matériau de support dont la taille va de 0,1 à 1 mm, ledit matériau de support étant un matériau hydrophobe dans le cas d'un médicament ayant une solubilité dans l'eau de 1 mg/ml ou plus et ledit matériau de support étant un matériau hydrophile dans le cas d'un médicament ayant une solubilité dans l'eau inférieure à 1 mg/ml ;
(2) une matrice dans laquelle lesdites granules sont incorporées, ladite matrice comprenant un polymère gonflable et désagrégeable et un matériau de régulation du gonflement ; et
(3) une couche de modification de la libération comprenant un polymère hydrophobe de modification de la libération, un polymère hydrophile de modification de la libération, un polymère pH-dépendant de modification de la libération ou un mélange de ceux-ci.

2. Préparation orale à libération contrôlée selon la revendication 1, dans laquelle 50 à 100% du médicament sont présents à l'intérieur des granules, et le médicament restant se trouve à l'intérieur de la matrice ou de la couche de modification de la libération, ou à l'intérieur de la matrice et de la couche de modification de la libération sous une forme directement dispersée.

3. Préparation orale à libération contrôlée selon la revendication 1, dans laquelle le médicament a une solubilité dans l'eau dans la gamme allant de 1 mg/ml à 100 mg/ml, et les granules contenant le médicament sont préparées par granulation par voie humide.

4. Préparation orale à libération contrôlée selon la revendication 1, dans laquelle le médicament a une solubilité dans l'eau d'au moins 100 mg/ml, et les granules contenant le médicament sont préparées sous forme granulaire par dispersion du médicament lors de la fusion des composants des granules.

5. Préparation orale à libération contrôlée selon la revendication 1, dans laquelle le médicament a une solubilité dans l'eau inférieure à 1 mg/ml, et les granules contenant le médicament sont préparées par un procédé de dispersion de corps solides.

6. Préparation orale à libération contrôlée selon la revendication 1, dans laquelle le matériau hydrophobe est au moins un matériau sélectionné dans le groupe constitué des acides gras, des esters d'acides gras, des alcools d'acides gras, des mono-, di- et triglycérides d'acide gras, des cires, de l'huile de ricin hydrogénée et de l'huile végétale hydrogénée.

7. Préparation orale à libération contrôlée selon la revendication 6, dans laquelle l'alcool d'acide gras est au moins un alcool sélectionné dans le groupe constitué de l'alcool cétostéarylique, l'alcool stéarylique, l'alcool laurylique et l'alcool myristylique , l'ester d'acide gras est au moins un ester sélectionné dans le groupe constitué du glycéryl monostéarate, du glycérol monostéarate, du monoglycéride acétylé, de la tristéarine, de la tripalminitine, de la cire de cetyl ester, du glycéryl palmitostéarate et du glycéryl béthanate ; et la cire est au moins une cire sélectionnée dans le groupe constitué de la cire d'abeille, de la cire de carnauba, de la glyco-cire et de la cire de ricin.

8. Préparation orale à libération contrôlée selon la revendication 1, dans laquelle le matériau hydrophile est au moins un matériau sélectionné dans le groupe constitué de polyalcoylène glycol et d'un polymère hydrophile carboxyvinylique, et le médicament est dispersé sous forme solide dans ledit polymère hydrophile.

9. Préparation orale à libération contrôlée selon la revendication 1, dans laquelle le polymère gonflable et désagrégeable est au moins un polymère sélectionné dans le groupe constitué de la hydroxypropylcellulose, la hydroxypropylméthylcellulose, de l'oxyde de polyéthylène, de l'alginate de sodium, de la povidone, de l'alcool polyvinylique et de la carboxyméthylcellulose de sodium.

10. Préparation orale à libération contrôlée selon la revendication 1, dans laquelle ledit matériau de régulation de gonflement est au moins un matériau sélectionné dans le groupe constitué de la carboxyméthylcellulose de sodium réticulée et de la polyvinylpyrrolidone réticulée.

11. Préparation orale à libération contrôlée selon la revendication 1, dans laquelle ledit polymère hydrophobe de modification de la libération qui est utilisé pour la formation de la couche de modification de la libération est au moins un polymère sélectionné dans le groupe constitué d'éthylcellulose, de gomme-laque, d'ammonio méthacrylate copolymère ; ledit polymère hydrophile de modification de la libération est au moins un polymère sélectionné dans le groupe constitué d'hydroxyalkylcellulose et d'hydroxypropylalkylcellulose ; et ledit polymère pH-dépendant de modification de la libération est au moins un polymère sélectionné dans le groupe constitué d'hydroxyalkylcellulose phthalate, d'hydroxyalkylméthylcellulose phthalate, de cellulose acétyl phthalate, de sodium cellulose acétate phthalate, de cellulose ester phthalate, de cellulose éther phthalate, et du copolymère anionique d'acide méthacrylique avec du méthyl ou de l'éthyl méthacrylate.

12. Préparation orale à libération contrôlée selon la revendication 1, dans laquelle ladite couche de modification de la libération constitue 1 à 20% en poids rapporté au poids total de la matrice, et les granules contenant le médicament atteignent 50 à 80% en poids rapporté au poids total de la préparation.

13. Préparation orale à libération contrôlée selon la revendication 1, dans laquelle le médicament est sélectionné dans le groupe suivant :
les agents thérapeutiques destinés à l'aconurésie, sélectionnés parmi l'oxybutynine et la tolérodine ;
les bloqueurs de canaux calcium sélectionnés parmi la nifédipine, le verapamil, l'israpidine, la nilvadipine, la flunarizine, la nimodipine, le diltiazem, la nicardipine, la nisoldipine, la felodipin, l'amlodipine, la cinarizine et la pendiline ;
les antagonistes bêta-adrénergiques sélectionnés parmi le propadonol et le metoprolole ;
les inhibiteurs de l'enzyme de conversion de l'angiotensine sélectionnés parmi le captopril, l'enalaplil, le ramipril, le fosinopril, l'altiopril, le benazepril, I libenzapril, l'alacepril, le cilazapril, le cilazaprilat, le perindopril, le zofedopril, le lisinopril, l'imidapril, le spirapril, le rentiapril, le delapril, l'alindapril, l'indalapril et le quinalapril ;
les agents anti-inflammatoires non stéroïdaux sélectionnés parmi le ketorolac, le ketoprofène, le benoxaprofène, le caprofène, le flubiprofène, le fenoprofène, le suprofène, le fenbufène, l'ibuprofène, l'indoprofène, le naproxène, le miroprofène, l'oxaproïne, le pranoprofène, le pirprofène, l'acide thiaprofénique, le fluprofène, l'alminoprofène, l'acide bucloxique, l'alclofenac, l'acernaticine, l'aspirine, l'indométhacine, l'ibufenac, l'isoxepac, le profenac, le fentiazac, le clidanac, l'oxpinac, le sulindac, le tolmétine, le zomepirac, le zidométacine, le tenclofenac, le tiopinac, l'acide méfènamique, l'acide flufénamique, l'acide niflumique, l'acide méclofénamique, l'acide tolfénamique, le diflufenisal, l'isoxicam et le sudoxicam ;
les agents thérapeutiques destinés aux troubles respiratoires, sélectionnés parmi la théophylline, le salbutamol, l'aminophylline, le dextrométorphane et la pseudoephedrine ;
les analgésiques sélectionnés parmi le tramadol, l'acetaminophène, la morphine, l'hydromorphone, l'oxycodone et le propoxyphène ;
les médicaments psycho-neuronaux sélectionnés parmi la fluoxetine, la paroxetine, le buspirone, la carmabazepine, le carvidopa, le levodopa, le méthylphénidate, le trazodone, l'acide valproïque, l'amitriptyline, la carbamazepine, l'ergoloïde, l'haloperidol et le lorazepam ;
les antibiotiques sélectionnés parmi le dihydrate d'azithromycine, les antibiotiques du groupe des céphalosporines, la clarithromycine, la doxycycline et la nitrofurantonine ;
les agents antihyperlipidémiants sélectionnés parmi le bezafibrate, le fenofibrate, l'éthofibrate et la lovastatine ;
les agents antidiabétiques sélectionnés parmi la glyburide, la glipizide et la metformine ; et
la cyclobenzaprine, la favotidine, la nizatidine, le propafénone, le clonazepam, la hyoscyamine, la diphénhydramine, l'orlistat et la doxazosine.

14. Préparation orale à libération contrôlée selon la revendication 1, dans laquelle le médicament est libéré suivant une libération d'ordre zéro sur au moins 8 à 24 heures lors de l'administration de la préparation.

15. Préparation orale de libération contrôlée selon la revendication 1, dans laquelle la dégradation de la surface de la matrice provoque la libération de 0 à 20% de la quantité totale de granules sur 0 à 4 heures, de 0 à 50% sur 0 à 8 heures, de 0 à 70% sur 0 à 16 heures, et de 0 à 100% sur 0 à 24 heures.
